(19) **Europäisches Patentamt**
**European Patent Office**
**Office européen des brevets**

(11) **EP 2 382 971 A1**

(12) **EUROPEAN PATENT APPLICATION**
published in accordance with Art. 153(4) EPC

(43) Date of publication:
**02.11.2011 Bulletin 2011/44**

(51) Int Cl.:
*A61K 9/30* (2006.01)   *A61K 47/02* (2006.01)
*A61K 47/20* (2006.01)   *A61K 47/22* (2006.01)
*A61K 47/46* (2006.01)

(21) Application number: **10735924.2**

(22) Date of filing: **29.01.2010**

(86) International application number:
**PCT/JP2010/051294**

(87) International publication number:
**WO 2010/087462 (05.08.2010 Gazette 2010/31)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 JP 2009018677**

(71) Applicant: **Dainippon Sumitomo Pharma Co., Ltd. Osaka 541-8524 (JP)**

(72) Inventors:
• **MATONO, Mitsuhiro**
  **Ibaraki-shi**
  **Osaka 567-0878 (JP)**

• **KOBAYASHI, Hirohisa**
  **Ibaraki-shi**
  **Osaka 567-0878 (JP)**
• **IKEDA, Yuki**
  **Ibaraki-shi**
  **Osaka 567-0878 (JP)**
• **OCHIAI, Yasushi**
  **Ibaraki-shi**
  **Osaka 567-0878 (JP)**

(74) Representative: **Webster, Jeremy Mark et al**
**Mewburn Ellis LLP**
**33 Gutter Lane**
**London**
**EC2V 8AS (GB)**

(54) **ORALLY DISINTEGRATING TABLET HAVING INNER CORE**

(57)   The present invention provides an orally disintegrating tablet with improved photostability of a medicament unstable to light. The orally disintegrating tablet has an inner core and an outer layer that covers the surface of the inner core, wherein the inner core contains the medicament unstable to light and the outer layer contains a light-absorbing substance such as Red No.2, Red No. 3, Yellow No.4, Yellow No.5, Blue No.1, Red No.3 aluminum lake, Yellow No.4 aluminum lake, Yellow No.5 aluminum lake, Blue No.1 aluminum lake, Blue No.2 aluminum lake, red ferric oxide, yellow ferric oxide, black iron oxide, carmine, or sodium copper chlorophyllin.

**Description**

Technical Field

The present invention relates to an orally disintegrating tablet that ensures improved photostability of a medicament that is unstable to light.

Background Art

Due to recent rapid aging of the population, an increasing number of elderly people suffer from deterioration or disorders of ingestion functions (mastication, deglutition, etc.) caused by a decrease in various physiological functions or senile dementia. These elderly patients often have difficulties upon oral administration of tablet medicament. At the same time, fast-paced modern society has been demanding development of orally disintegrating tablets that are compact, mobile, and stable upon ingestion, allowing people to easily ingest them at any time and in any place. Further, in terms of the recent trend toward setting the plurality of medicaments in a single package, it is critical to ensure stability of the medicament after opening, and photostability of orally disintegrating tablets.

Heretofore, various methods have been employed to ensure medicament stability when preparing medicaments unstable to light. For example, Patent Document 1 discloses a nifedipine soft capsule obtained by encapsulating nifedipine in a soft capsule having a coloring agent dispersed therein, thus preventing photo-degradation or deterioration of nifedipine unstable to light. However, the techniques disclosed in Patent Document 1 are all attained by incorporating a coloring agent into the capsule encapsulating the medicament unstable to light; thus, it is difficult to apply these techniques to orally disintegrating tablets.

Patent Document 2 discloses photostable tablets obtained by coating dihydropyridine derivative tablets with a film containing ferric oxide. However, this technique merely discloses the effect obtained by a combination of titanium oxide having light-blocking properties and ferric oxide. Titanium oxide has photocatalytic action and may facilitate degradation by light for some medicaments. Further, this technique involving coating of medicament requires extra manufacturing steps, which greatly increase time and labor, thereby increasing the cost. Further, when the entire pharmaceutical preparation is coated, rapid disintegrability and rapid solubility of the medicament in the oral cavity are impaired, and the preparation thereby fails to express functionality as an orally disintegrating tablet.

Further, as one of the methods to ensure photostability of uncoated tablets, granules, fine grain agents, or powder medicaments, Patent Document 3 discloses a composition with improved photostability obtained by incorporating one or more substances selected from yellow and red coloring agents in a lipophilic medicament unstable to light. However, these methods for improving photostability are still insufficient to ensure a desired photostabilizing effect.

On the other hand, in the field of dry-coating technology, Patent Document 4 discloses a rapidly soluble disintegrating formulation. However, Patent Document 4 nowhere discloses photostability. Further, Patent Document 5 discloses further coating dry-coated tablets with titanium oxide, or the like, to ensure photostability; however, eventually this method also ensures photostability by coating with film, thereby impairing functionality as an orally disintegrating tablet. As such, so far, there is no successful development of an orally disintegrating tablet in which the inherent poor photostability of the medicament is sufficiently improved.

Citation List

Patent Document

[Patent Document 1] Japanese Unexamined Patent Publication No. 1980-22645
[Patent Document 2] Japanese Unexamined Patent Publication No. 2003-104888
[Patent Document 3] Japanese Unexamined Patent Publication No. 2000-7583
[Patent Document 4] Pamphlet of International Publication 2003/028706
[Patent Document 5] Japanese Unexamined Patent Publication No. 2007-91648

Summary of Invention

Technical Problem

An object of the present invention is to provide an orally disintegrating tablet that ensures improved photostability of a medicament unstable to light.

Solution to Problem

In order to solve the above objective, the inventors of the present invention focused on dry-coating technology. More specifically, the inventors conceived of incorporating a medicament unstable to light only in the inner core of a tablet and covering the core with an outer layer, thereby improving photostability of the medicament unstable to light. However, as shown in the later-described Test Example, the inventors found that the photo-degradation of a medicament unstable to light may not always be suppressed only by incorporating the medicament unstable to light in the inner core (for example, see Comparative Example 3). Therefore, the inventors then added titanium oxide, which is widely used to ensure photostability in film coating technology, to the outer layer of the dry-coated tablet; however, it was revealed that decomposition of a medicament unstable to light cannot be suppressed (for example, see Comparative Example 4).

The inventors of the present invention conducted extensive research, and, surprisingly, found that, by incorporating a medicament unstable to light in the inner core and incorporating a light-absorbing substance in an outer layer, it is possible to desirably increase the photostability of the medicament unstable to light and almost completely suppress the decomposition. Further, it was also revealed that, by incorporating a certain amount or more of the light-absorbing substance in the outer layer regardless of the concentration of the light-absorbing substance in the outer layer and the thickness of the outer layer, the decomposition of the medicament unstable to light was almost completely suppressed even without combination of titanium oxide, or the like, which serves as a light-blocking substance. Further, the inventors also confirmed that the obtained preparation ensures functionality as an orally disintegrating tablet. Thereby, the inventors completed the present invention.

Specifically, the present invention relates to the following aspects.

[1] An orally disintegrating tablet comprising an inner core and an outer layer that covers a surface of the inner core, wherein the inner core contains a medicament unstable to light and the outer layer contains a light-absorbing substance.

[2] The orally disintegrating tablet according to [1], wherein the light-absorbing substance is at least one member selected from the group consisting of Food Red No.2, Food Red No.3, Food Red No.102, Food Red No.104, Food Red No.105, Food Red No.106, Food Yellow No.4, Food Yellow No.5, Food Green No.3, Food Blue No.1, Food Blue No.2, Food Red No.3 aluminum lake, Food Yellow No.4 aluminum lake, Food Yellow No.5 aluminum lake, Food Blue No.1 aluminum lake, Food Blue No.2 aluminum lake, red ferric oxide, yellow ferric oxide, black iron oxide, yellow oxide of iron, carmine, sodium copper chlorophyllin, copper chlorophyll, and red iron oxide.

[3] The orally disintegrating tablet according to [1], wherein the light-absorbing substance is at least one member selected from the group consisting of Food Red No.2, Food Red No.3, Food Red No.102, Food Red No.104, Food Red No.105, Food Red No.106, Food Yellow No.4, Food Yellow No.5, Food Green No.3, Food Red No.3 aluminum lake, Food Yellow No.4 aluminum lake, Food Yellow No.5 aluminum lake, red ferric oxide, yellow ferric oxide, black iron oxide, yellow oxide of iron, carmine, sodium copper chlorophyllin, copper chlorophyll, and red iron oxide.

[4] The orally disintegrating tablet according to [1], wherein the light-absorbing substance is at least one member selected from the group consisting of Food Red No.2, Food Red No.3, Food Yellow No.4, Food Yellow No.5, Food Blue No.1, Food Red No.3 aluminum lake, Food Yellow No.4 aluminum lake, Food Yellow No.5 aluminum lake, Food Blue No.1 aluminum lake, Food Blue No.2 aluminum lake, red ferric oxide, yellow ferric oxide, black iron oxide, carmine, and sodium copper chlorophyllin.

[5] The orally disintegrating tablet according to any one of [1] to [4], wherein the light-absorbing substance is at least one member selected from the group consisting of red ferric oxide, yellow ferric oxide, and black iron oxide.

[6] The orally disintegrating tablet according to any one of [1] to [5], wherein the outer layer contains the light-absorbing substance in an amount of 0.01 to 600 $\mu g/mm^2$ per unit surface area of the inner core.

[7] The orally disintegrating tablet according to any one of [1] to [5], wherein the outer layer contains the light-absorbing substance in an amount of 0.1 to 150 $\mu g/mm^2$ per unit surface area of the inner core.

[8] The orally disintegrating tablet according to any one of [1] to [5], wherein the outer layer contains the light-absorbing substance in an amount of 0.2 to 20 $\mu g/=mm^2$ per unit surface area of the inner core.

[9] The orally disintegrating tablet according to any one of [1] to [5], wherein the outer layer contains the light-absorbing substance in an amount of 1.5 to 10 $\mu g/=mm^2$ per unit surface area of the inner core.

[10] A method for manufacturing an orally disintegrating tablet comprising an inner core containing a medicament unstable to light and an outer layer that covers a surface of the inner core, the method comprising the steps of: molding inner-core-forming powder containing the medicament unstable to light to form a core; supplying the core into a die of a dry-coated tabletting machine supplied in advance with outer-layer-forming powder containing a light-absorbing substance; and further supplying outer-layer-forming powder and compression-molding the whole powder.

[11] A method for manufacturing an orally disintegrating tablet comprising an inner core containing a medicament unstable to light and an outer layer that covers a surface of the inner core using a compression molding means having an upper punch and a lower punch, which are arranged in the vertical direction of a die, both the upper punch and the lower punch having a double structure comprising a center punch and an outer punch surrounding the outer periphery of the center punch, and being slidable and capable of a compressing operation; the method comprising : a first outer-layer supplying step of supplying outer-layer-forming powder containing a light-absorbing substance into a space above the lower center punch and surrounded by the lower outer punch; an inner-core supplying step of supplying inner-core-forming powder containing a medicament unstable to light into a space surrounded by the lower outer punch and above the outer-layer-forming powder supplied in the outer-layer supplying step; an outer layer and inner-core molding step of compression-molding the outer-layer-forming powder and the inner-core-forming powder supplied in the outer-layer supplying step and the inner-core supplying step; a second outer-layer supplying step of supplying the outer-layer-forming powder onto the molded product in the die formed in the outer layer and inner-core molding step and into a surrounding space thereof; and a whole molding step of compression-molding the molded product and the outer-layer-forming powder.

[12] A method for manufacturing an orally disintegrating tablet comprising an inner core containing a medicament unstable to light and an outer layer that covers a surface of the inner core using a compression molding means having an upper punch and a lower punch, which are arranged in the vertical direction of a die, both the upper punch and the lower punch having a double structure comprising a center punch and an outer punch surrounding the outer periphery of the center punch, and being slidable and capable of a compressing operation; the method comprising: an inner-core supplying step of supplying inner-core-forming powder containing a medicament unstable to light into a space above the lower center punch and surrounded by the lower outer punch; an inner-core molding step of compression-molding the inner-core-forming powder supplied in the inner-core supplying step; and an outer-layer supplying step of supplying outer-layer-forming powder onto the molded product in the die formed in the inner-core molding step and into a surrounding space thereof until a tip of the lower center punch finally takes a position protruding from a tip of the lower outer punch; and a whole molding step of compression-molding the molded product and the outer-layer-forming powder with the tips of the lower outer punch and the lower center punch aligned with each other.

[13] A method for improving photostability of a medicament unstable to light contained in an orally disintegrating tablet, the method comprising forming the orally disintegrating tablet with an inner core and an outer layer that covers the surface of the inner core; incorporating the medicament into the inner core; and incorporating a light-absorbing substance into the outer layer.

[14] A method for improving photostability of a medicament unstable to light contained in an orally disintegrating tablet that comprises an inner core containing the medicament and an outer layer that covers the surface of the inner core, the method comprising incorporating a light-absorbing substance into the outer layer.

[15] A method for stabilizing a medicament unstable to light, the method comprising coating a tablet containing the medicament with an outer layer containing a light-absorbing substance.

[Effect of the Invention]

The present invention provides an orally disintegrating tablet that ensures improved photostability of a medicament unstable to light in a very simple manner. This enables quality maintenance of the easily administrable medicinal preparations such as orally disintegrating tablets comprising medicaments which are unsuitable for coating. The present invention thus makes it possible to provide a photostable orally disintegrating tablet that enables patients having difficulties in deglutition, such as elderly patients, or busy working people to more easily ingest the tablet in any situation.

[Mode for carrying out the invention]

The orally disintegrating tablet of the present invention comprises an inner core and an outer layer that covers the surface of the inner core, wherein the inner core contains a medicament unstable to light and the outer layer contains a light-absorbing substance, the orally disintegrating tablet being rapidly dissolvable or disintegratable in the oral cavity upon administration without drinking water; it is also administrable with water, as general preparations are administered.

The dissolution or disintegration time of the orally disintegrating tablet of the present invention in the oral cavity is generally within 1 minute, preferably within 45 seconds, and more preferably within 30 seconds. Further, the hardness of the orally disintegrating tablet as an absolute hardness is preferably not less than 0.5 $N/mm^2$, more preferably not less than 1.0 $N/mm^2$. The "absolute hardness" is an absolute hardness of each tablet per unit area, which is found according to the hardness/sectional area of half of a tablet. For example, for a tablet (flat tablet) having a flat upper surface and a flat lower surface, the absolute hardness is found as follows using hardness measured with a tablet hardness tester and tablet cross section area (diameter $\times$ thickness).

$$\text{Absolute hardness } [N/mm^2] = \text{hardness}[N] \div (\text{tablet diameter }[mm] \times \text{tablet thickness }[mm])$$

In the present invention, "medicament unstable to light" can be defined in the following manner. An uncoated tablet containing 5 wt% of the medicament, which does not contain photostabilizing substances, such as light-absorbing substances or light-blocking substances (not including, however, dry-coated tablets, such as those of the present invention), was exposed to light having a total illuminance of 1200000 lux• hr and a total near-ultraviolet radiation energy of 200 W•hr/m$^2$ according to "STABILITY TESTING: PHOTOSTABILITY TESTING OF NEW DRUG SUBSTANCES AND PRODUCTS" (Recommended for Adoption at Step 4 of the ICH Process on 6 November 1996 by the ICH Steering Committee). Then, "medicament unstable to light" is determined when a decomposition product and a decrease of 1.0% or more in its content are observed, or when any changes in appearance of the surface of uncoated tablet are observed as, for example, a value of 3 or more for $\Delta E$, which is a color difference value measured according to the L*a*b* color system using a spectral color-difference meter.
Examples of medicaments unstable to light include, but are not limited to, medicaments having dihydropyridine structures, such as nifedipine, nisoldipine, nitrendipine, benidipine, manidipine, barnidipine, efonidipine, or nilvadipine; newquinolone medicaments, such as norfloxacin, ofloxacin, lomefloxacin or sparfloxacin; and vitamin preparations, such as Vitamins A, B2, B6, B12, D, or K.

In the present invention, "light-absorbing substance" indicates a substance having functionality to exhibit colors other than white by absorption of wavelengths involved in photo-degradation of a medicament. More specifically, "light-absorbing substance" may be any coloring agents or pigments, other than white pigments usable as medicinal additives. Examples thereof include Food Red No.2, Food Red No. 3, Food Red No.102, Food Red No.104, Food Red No.105, Food Red No.106, Food Yellow No.4, and Food Yellow No.5, Food Green No.3, Food Blue No.1, Food Blue No.2, Food Red No. 3 aluminum lake, Food Yellow No.4 aluminum lake, Food Yellow No.5 aluminum lake, Food Blue No.1 aluminum lake, Food Blue No.2 aluminum lake, red ferric oxide, yellow ferric oxide, black iron oxide, yellow oxide of iron, carmine, sodium copper chlorophyllin, copper chlorophyll, and red iron oxide. These can be used singly or in combination of two or more. Among them, preferable are Food Red No.2, Food Red No. 3, Food Red No.102, Food Red No.104, Food Red No.105, Food Red No.106, Food Yellow No.4, Food Yellow No.5, Food Green No.3, Food Red No. 3 aluminum lake, Food Yellow No.4 aluminum lake, Food Yellow No.5 aluminum lake, red ferric oxide, yellow ferric oxide, black iron oxide, yellow oxide of iron, carmine, sodium copper chlorophyllin, copper chlorophyll, and red iron oxide. These can be used singly or in combination of two or more. Further, yellow ferric oxide, black iron oxide, and red ferric oxide are more preferable. These can be used singly or in combination of two or more. Moreover, yellow ferric oxide and red ferric oxide, individually or in combination of two, are further preferable. In addition to these light-absorbing substances, it is possible to add a light-blocking substance to an extent not impairing the effect of the present invention. In the present invention, "light-blocking substance" indicates a substance that does not transmit irradiated light when the substance is compressed into a 0.5-mm-thick tablet, and which appears substantially white to human eyes. Examples thereof include titanium oxide, talc, and kaolin.

The particle diameter of the light-absorbing substance is not particularly limited insofar as it can be uniformly dispersed and incorporated in a solid oral composition as a medicinal product. For example, the particle diameter is set so that the mean volume diameter is 0.01 to 1.0 $\mu$m, preferably 0.01 to 0.5 $\mu$m, more preferably 0.1 to 0.5 $\mu$m, when measured with a laser diffraction/scattering particle-size distribution analyzer.

As described, the orally disintegrating tablet of the present invention contains an inner core comprising a medicament unstable to light, and an outer layer comprising the above-described light-absorbing substance. An appropriate content of the light-absorbing substance in the outer layer differs depending on the type of light-absorbing substance to be contained, the surface area of the inner core, and the thickness of the outer layer. However, for example, the content is preferably 0.001 to 40 mg, more preferably 0.005 to 15 mg, more preferably 0.01 to 10 mg, more preferably 0.02 to 4 mg, more preferably 0.02 to 2 mg, more preferably 0.03 to 1 mg, particularly preferably 0.1 to 0.7 mg, when the diameter of the inner core of the tablet is about 6 mm, the thickness of the inner core of the tablet is about 1 mm, the diameter of the outer layer of the tablet is about 8 mm, and the thickness of the outer layer is about 1 mm.

More specifically, the preferable content of the light-absorbing substance in the outer layer is 0.01 to 600 $\mu$g/=mm$^2$, more preferably 0.06 to 200 $\mu$g/mm$^2$, more preferably 0.1 to 150 $\mu$g/=mm$^2$, more preferably 0.2 to 60 $\mu$g/=mm$^2$, more preferably 0.2 to 20 $\mu$g/mm$^2$, more preferably 0.5 to 15 $\mu$g/=mm$^2$, and particularly preferably 1.5 to 10 $\mu$g/=mm$^2$, per unit surface area of the inner core. The content of the light-absorbing substance in the outer layer per unit surface area of the inner core is calculated by dividing the whole content of the light-absorbing substance in the outer layer by the surface area of the inner core.

The content of the medicament unstable to light in the inner core of the orally disintegrating tablet of the present invention is not particularly limited. The content of the medicament in the inner core can be 0.001 to 100 wt%.

The weight of the inner core of the orally disintegrating tablet of the present invention is 10 mg to 400 mg, more preferably 20 mg to 300 mg, and further preferably 50 mg to 150 mg. The weight of the outer layer of the orally disintegrating tablet is 50 mg to 800 mg, more preferably 70 mg to 600 mg, and further preferably 100 mg to 300 mg. For example, the weight ratio of the inner core to the outer layer (inner core/outer layer) is generally 0.05 to 2, preferably 0.2 to 1, and more preferably 0.3 to 0.5. The thickness of the outer layer is generally 0.3 mm to 2 mm, more preferably 0.5 mm to 1.5 mm.

In addition to the medicament unstable to light contained in the inner core and the light-absorbing substance contained in the outer layer, the inner core and the outer layer may individually contain other typical known ingredients used for orally disintegrating tablets, for example, excipients, disintegrating agents, binders, sweetening agents, perfume, taste correctives and/or odor correctives, and lubricants. The ingredients of the inner core and the outer layer may be the same or different. The inner core may have smaller strength.

In the present invention, examples of excipients include sorbitol, mannitol, maltitol, reducing starch sugar, xylitol, trehalose, glucose, refined sugar, lactose hydrate, calcium sulfate, calcium carbonate, reducing palatinose, erythritol, anhydrous dibasic calcium phosphate, and crystalline cellulose. They may be used in combination of two or more at appropriate ratios.

Examples of disintegrating agents include starch, carboxymethylcellulose, sodium carboxymethylcellulose, calcium carboxymethylcellulose, low-substituted hydroxypropylcellulose, sodium carboxymethylstarch, low-substituted sodium carboxymethylstarch, croscarmellose sodium, and crospovidon. They may be used in combination of two or more at appropriate ratios. "Starch" includes various kinds of starches derived from natural starches usable for medicinal products. Examples thereof include potato starch, corn starch, wheat starch, rice starch, soluble starch, partially-pregelatinized starch, pregelatinized starch, and hydroxypropylated starch.

Examples of binders include starch (including partially -pregelatinized starch), agar, dextrin, pullulan, hydroxyethylcellulose, hydroxyethylmethylcellulose, hydroxypropylcellulose, hydroxypropylmethylcellulose, polyvinylpyrrolidone, gelatin, methylcellulose, ethylcellulose, carboxymethylethylcellulose, carmellose sodium, gum arabic, gum arabic powder, polyvinyl alcohol, and alkylhydroxyethylcellulose. They may be used in combination of two or more at appropriate ratios.

Examples of sweetening agents include aspartame, acesulfam K, saccharin, saccharin sodium, stevia, sucralose, thaumatin, and neotame. They may be used in combination of two or more at appropriate ratios.

Examples of perfume include peppermint, spearmint, menthol, lemon, orange, grapefruit, pineapple, fruit, and yogurt. They may be used in combination of two or more at appropriate ratios.

Examples of taste correctives and/or odor correctives, include various amino acids and their salts thereof, such as sodium aspartate, alanine, arginine, glycine, glutamine, or glutamic acid; organic acids, such as adipic acid, ascorbic acid, citric acid, succinic acid, tartaric acid, or malic acid; sweetroot; triethyl citrate; taurine; and tannic acid. They may be used in combination of two or more at appropriate ratios.

Examples of lubricants include stearic acid, metal stearate, sodium stearyl fumarate, sucrose fatty acid ester, talc, hardened oil, and macrogol.

In addition to the above ingredients, the solid oral composition of the present invention may contain nontoxic and inactive additives generally used in the field of medicinal preparations. Examples thereof include general additives for medicinal products that do not substantially impair the effect of the present invention, such as coloring agents, fluidizers, surfactants, adsorbents, preservatives, stabilizers, wetting agents, antistatic agents, and pH adjusters.

The dry-coated tablet of the present invention may be manufactured using known methods. For example, it may be manufactured by molding in advance (for example, at 1 to 5 kN) inner-core-forming powder containing a medicament unstable to light to form a core using a tabletting machine, by supplying the resulting core in the die of a dry-coat tabletting machine in which the outer-layer-forming powder is already supplied, and then by supplying the outer-layer-forming powder, and finally by compression-molding the whole content in the die (for example, at 5 to 15 kN).

Further, for example, the dry-coated tablet of the present invention may be manufactured according to the method disclosed in Patent Document 6, such as a method for manufacturing an orally disintegrating tablet having an inner core containing a medicament unstable to light and an outer layer that covers a surface of the inner core using a compression molding means having an upper punch and a lower punch, which are arranged in the vertical direction of a die, both the upper punch and the lower punch having a double structure comprising a center punch and an outer punch surrounding the outer periphery of the center punch, and being slidable and capable of a compressing operation; the method comprising : a first outer-layer supplying step of supplying outer-layer-forming powder containing a light-absorbing substance into a space above the lower center punch and surrounded by the lower outer punch; an inner-core supplying step of supplying inner-core-forming powder containing a medicament unstable to light into a space surrounded by the lower outer punch and above the outer-layer-forming powder supplied in the outer-layer supplying step; an outer layer and inner-core molding step of compression-molding the outer-layer-forming powder and the inner-core-forming powder supplied in the outer-layer supplying step and the inner-core supplying step; a second outer-layer supplying step of supplying the outer-layer-forming powder onto the molded product in the die formed in the outer layer and inner-core molding step and into a surrounding space thereof; and a whole molding step of compression-molding the molded product and the outer-layer-forming powder.

The tablet may also be manufactured by a method for manufacturing an orally disintegrating tablet comprising an inner core containing a medicament unstable to light and an outer layer that covers a surface of the inner core using a compression molding means having an upper punch and a lower punch, which are arranged in the vertical direction of a die, both the upper punch and the lower punch having a double structure comprising a center punch and an outer punch surrounding the outer periphery of the center punch, and being slidable and capable of a compressing operation; the method comprising: an inner-core supplying step of supplying inner-core-forming powder containing a medicament unstable to light into a space above the lower center punch and surrounded by the lower outer punch; an inner-core molding step of compression-molding the inner-core-forming powder supplied in the inner-core supplying step; and an outer-layer supplying step of supplying outer-layer-forming powder onto the molded product in the die formed in the inner-core molding step and into a surrounding space thereof until a tip of the lower center punch finally takes a position protruding from a tip of the lower outer punch; and a whole molding step of compression-molding the molded product and the outer-layer-forming powder with the tips of the lower outer punch and the lower center punch aligned with each other.

[Patent Document 6] Pamphlet of International Publication 2005/046978

It is also possible to granulate the components of the inner core or the outer layer in advance, and then mix the components. Examples of the granulation methods include extrusion granulation, pulverizing granulation, dry compaction granulation, fluid-bed granulation, tumbling granulation, tumbling fluid-bed granulation, and high-speed agitating granulation. Examples of tabletting methods for the inner core or outer layer include wet tabletting and direct tabletting.

Examples

The present invention is more specifically explained below in reference to the Examples and Comparative Examples. The present invention is, however, not limited to those examples. In the explanation below, "%" means "wt%", unless otherwise specified.

[Experiment 1]

Tablet Formulation (mg)

[Table 1]

| | | Dry-Coated Tablets | | | General Tablets | | Dry-Coated Tablets | |
|---|---|---|---|---|---|---|---|---|
| | | Example 1 | Example 2 | Example 3 | Comparative Example 1 | Comparative Example 2 | Comparative Example 3 | Comparative 4 |
| Light-Absorbing Substance or Light- Blocking Substance | | Yellow ferric oxide | Yellow ferric oxide | Yellow ferric oxide | - | Yellow ferric oxide | - | Titanium Oxide |
| Content in Outer Layer | | 0.1% | 1.5% | 3.0% | - | 3.0% (Based on the Entire Formulation) | - | 15% |
| Content in Outer Layer per Surface Area of Inner Core ($\mu$g/Mm$^2$) | | 1.79 | 26.87 | 53.74 | - | - | - | 268.71 |
| Inner Core | D-Mannitol | 35.55 | 35.55 | 35.55 | 157.05 | 152.19 | 35.55 | 35.55 |
| | Corn Starch | 3.95 | 3.95 | 3.95 | 17.45 | 16.91 | 3.95 | 3.95 |
| | Nifedipine | 10 | 10 | 10 | 10 | 10 | 10 | 10 |
| | Yellow ferric oxide | - | - | - | - | 4.05 | - | - |
| | Magnesium Stearate | 0.5 | 0.5 | 0.5 | 0.5 | 1.85 | 0.5 | 0.5 |
| | Total | 50 | 50 | 50 | 185 | 185 | 50 | 50 |
| Outer Layer | D-Mannitol | 120.16 | 118.46 | 116.64 | - | - | 120.29 | 102.06 |
| | Corn Starch | 13.35 | 13.16 | 12.96 | - | - | 13.37 | 11.34 |
| | Titanium Oxide | - | - | - | - | - | - | 20.25 |
| | Yellow ferric oxide | 0.14 | 2.03 | 4.05 | - | - | - | - |
| | Magnesium Stearate | 1.35 | 1.35 | 1.35 | - | - | 1.35 | 1.35 |
| | Total | 135 | 135 | 135 | | | 135 | 135 |
| Total | | 185 | 185 | 185 | 185 | 185 | 185 | 185 |

D-mannitol (Roquette)
Corn starch (XX16)W (Nihon Shokuhin Kako Co., Ltd)
Nifedipine (Wako Pure Chemical Industries, Ltd.)
Magnesium stearate (Taihei Chemical Industrial Co., Ltd.) Titanium oxide (Wako Pure Chemical Industries, Ltd.)
Yellow ferric oxide (Kishi Kasei Co., Ltd.)
In Examples 1 to 3 and Comparative Examples 3 and 4, according to the formulations of Table 1, the respective components of the inner core and the outer layer of dry-coated tablets were mixed well in respective mortars.

Preparation of inner core

The inner-core-forming powder in an amount for a single tablet formulation was compressed using a simple molding machine (TB-20H 20-kN table press, NPa SYSTEM CO., LTD.) at a pressure of 2 kN, thereby obtaining a tablet 6 mm in diameter. The thickness of the inner core was about 1 mm.

Preparation of outer layer

First, the bottom portion of the outer layer was formed. 50 mg of outer-layer-forming powder was compressed using a table press at a pressure of 2 kN, thereby obtaining a tablet 8 mm in diameter and 1 mm in thickness. Then, the inner core prepared above was disposed on the center of the produced bottom portion of the outer layer, and then 85 mg of outer-layer-forming powder was added to form the lateral and upper portions of the outer layer. The whole content in the die was compressed using a table press at a pressure of 10 kN. Thereby, a dry-coated tablet 8 mm in diameter and 3 mm in thickness having flat upper and lower surfaces was obtained.
In Comparative Examples 1 and 2, according to the formulations of Table 1, the respective components were mixed, and 185 mg of each resulting powder was compressed using a table press at a pressure of 10 kN, thereby obtaining a tablet 8 mm in diameter.

Test Example 1

A photostability test was performed with respect to the tablets obtained in Examples 1 to 3 and Comparative Examples 1 to 4.
The conditions used in light irradiation on the tablets are shown below. The tablets were irradiated with D65 daylight-color light (20-W, FLR20S-D-EDL-D65/M) for color comparison and testing at 3500 lux for eight days using a photostability tester (Nagano Science CO. LTD., Model: LT-120D3CJ).
After the light irradiation, quantitative analysis of the decomposition product (oxidant) of nifedipine in the tablets was performed by liquid chromatography (HPLC).
The changes in appearance of the tablets after light irradiation were examined by observing the changes in color by visual inspection. Table 2 shows the results.

■ Evaluation criteria

(1) Amount of generated oxidant

■ Sample preparation method

To prepare sample solutions, each tablet before light irradiation (the tablets kept in a dark place at 5°C after manufacture) and the same after light irradiation were placed individually in a 100-mL brown measuring flask. Also, as a standard solution, 10 mg of an active pharmaceutical ingredient (API) (nifedipine) was placed in a 100-mL brown measuring flask. 20 mL of water was added to each solution, and a 10-minute ultrasonic treatment was performed.
60 mL of 50%v/v acetonitrile in water heated to 40°C was added to each resulting solution and shaken for 30 minutes. Further, more mixed solution of 50%v/v acetonitrile in water was added to each solution in a measuring cylinder until the total volume was 100 mL, and ultrasonic treatment was performed again for 10 minutes. Each solution was placed in a 2-mL Eppendorf tube to be subjected to centrifuge at 15000 rpm for 10 minutes. The supernatant was used as a measurement sample for HPLC.

■ HPLC analysis conditions

Column: Supercosil LC-18-DB (150 × 4.6 mm, 3 μm)
Column temperature: 25°C

Wavelength: 270 nm
Mobile phase: 20% acetonitrile/30% methanol/50% 0.01 M TEAA Buffer*1) (a pH value = 5.0)
Flow rate: 0.75 mL/min
Injection rate: 50 μm
Syringe washing solution: water/acetonitrile mixed solution (1:1)
*1) TEAA Buffer (triethylamine-acetate buffer): obtained by adding acetic acid to 0.01 M triethylamine in water and adjusting pH to 5.0.

(2) Oral disintegration time

Oral disintegration time was measured with three healthy male test subjects using the obtained orally disintegrating tablets before light irradiation by finding an average value of durations (seconds) from when the tablets were placed in the oral cavities to when the tablets disintegrate in the oral cavities.

(3) Absolute hardness

Using a TH-203MP tablet hardness tester (Fuji-Sangyo Co. Ltd.), the hardness of each of the obtained orally disintegrating tablet before light irradiation was measured. The absolute hardness was found for each tablet according to the following equation.
$$\text{Absolute hardness } [N/mm^2] = \text{hardness } [N] \div (\text{tablet diameter } [mm] \times \text{tablet thickness } [mm])$$

Processing with a tablet packaging machine to set the plurality of medicaments in a single package requires an absolute hardness of 1 N/mm2 or more.
[Table 2]

| Sample | Increase (%) in Content of Oxidant after Light Irradiation | Change in Appearance | Disintegration Time (s) in the Oral Cavity | Absolute Hardness (N/=mm2) |
|---|---|---|---|---|
| Example 1 | 0.1 | Not Observed | 20 | 1.01 |
| Example 2 | 0.4 | Not Observed | - | - |
| Example 3 | 0.0 | Not Observed | - | - |
| Comparative Example 1 | 37.2 | Significant Change observed | - | - |
| Comparative Example 2 | 7.3 | Slight Change observed | - | - |
| Comparative Example 3 | 13.1 | Not Observed | 19 | 1.12 |
| Comparative Example 4 | 1.4 | Not Observed | 24 | 0.90 |

The oxidant increase rate is calculated according to the following: (the area percentage of oxidant in the tablet after light irradiation) - (area in percentage of oxidant in the tablet before light irradiation).
As shown in Table 2, the general tablets of Comparative Example 1 not subjected to photostabilizing treatment had significant changes in appearance, and the oxidant increase rate was very high. In contrast, the oxidant increase rate was relatively reduced in the tablets of Comparative Example 3, in which nifedipine was added only to the inner core of the dry-coated tablets, but the increase rate was still high. In the tablets of Comparative Example 4, in which the outer layer of the dry-coated tablet contains 15% titanium oxide, the oxidant increase rate was not fully desirable, although it was significantly low. The incorporation of 15% titanium oxide also had a tendency to cause a delay of disintegration and a decrease in tablet strength. On the other hand, in the preparations of Examples 1 to 3, in which the outer layer of the dry-coated tablet contains yellow ferric oxide, the oxidant increase rate was reduced almost completely, not to mention the changes in appearance. Further, since the oxidant increase rate was not reduced in the general tablets of Comparative Example 2 containing 3.0% yellow ferric oxide, which also had a significant change in appearance by visual inspection, it was revealed that it is important to incorporate yellow ferric oxide into the outer layer of the dry-coated tablet. Moreover, surprisingly, the oxidant increase rate was reduced almost completely by incorporating only 0.1% yellow ferric oxide in the outer layer of the dry-coated tablet (Example 1). This dry-coated tablet had a rapid oral disintegration time, i.e., 20 seconds, and a high absolute hardness, i.e., 1 N/mm$^2$ or more. Accordingly, this dry-coated tablet is adaptable to a tablet packaging machine to set the plurality of medicaments in a single package and is considered to be a very high-quality orally disintegrating tablet.

[Experiment 2]

Tablet formulation (mg)

[Table 3]

| | | Dry-Coated Tablets | | | | | | | | |
|---|---|---|---|---|---|---|---|---|---|---|
| | | Example 4 | Example 5 | Example 6 | Example 7 | Example 8 | Example 9 | Example 10 | Comparative Example 5 | Comparative Example 6 |
| Light-Absorbing Substance or Light-Blocking Substance | | Food Red No.2 | Food Yellow No.4 | Food Blue No.1 | Red ferric oxide | Black Iron Oxide | Red ferric oxide | Yellow Ferric Oxide | Talc | Kaolin |
| Content in Outer Layer | | 0.10% | 0.10% | 0.10% | 0.10% | 0.10% | 0.01% | 0.10% | 10% | 10% |
| Content in Outer Layer per Surface Area of Inner Core ($\mu$g/Mm$^2$) | | 1.79 | 1.79 | 1.79 | 1.79 | 1.79 | 0.18 | 1.79 | 179.14 | 179.14 |
| Inner Core | D-Mannitol | 35.55 | | | | | | - | 35.55 | |
| | Corn Starch | 3.95 | | | | | | - | 3.95 | |
| | Nifedipine | 10 | | | | | | 50 | 10 | |
| | Magnesium Stearate | 0.5 | | | | | | - | 0.5 | |
| | Total | 50 | | | | | | 50 | 50 | |

(continued)

| | | Dry-Coated Tablets | | | | | | | | |
|---|---|---|---|---|---|---|---|---|---|---|
| | | Example 4 | Example 5 | Example 6 | Example 7 | Example 8 | Example 9 | Example 10 | Comparative Example 5 | Comparative Example 6 |
| Outer Layer | D-Mannitol | 120.1635 | 120.1635 | 120.1635 | 120.1635 | 120.1635 | 120.1635 | 120.1635 | 108.135 | 108.135 |
| | Corn Starch | 13.3515 | 13.3515 | 13.3515 | 13.3515 | 13.3515 | 13.3515 | 13.3515 | 12.015 | 12.015 |
| | Food Red No.2 | 0.135 | - | - | - | - | - | - | - | - |
| | Food Yellow No.4 | - | 0.135 | - | - | - | - | - | - | - |
| | Food Blue No.1 | - | - | 0.135 | - | - | - | - | - | - |
| | Red ferric oxide | - | - | - | 0.135 | - | 0.0135 | - | - | - |
| | Black Iron Oxide | - | - | - | - | 0.135 | - | - | - | - |
| | Yellow ferric oxide | - | - | - | - | - | - | 0.135 | - | - |
| | Talc | - | - | - | - | - | - | - | 13.5 | - |
| | Kaolin | - | - | - | - | - | - | - | - | 13.5 |
| | Magnesium Stearate | 1.35 | 1.35 | 1.35 | 1.35 | 1.35 | 1.35 | 1.35 | 1.35 | 1.35 |
| | Total | 135 | 135 | 135 | 135 | 135 | 135 | 135 | 135 | 135 |
| Total | | 185 | 185 | 185 | 185 | 185 | 185 | 185 | 185 | 185 |

Food Red No.2 (Taketombo Co.)
Food Yellow No.4 (Taketombo Co.)
Food Blue No.1 (Taketombo Co.)
Red ferric oxide (Taketombo Co.)
Black iron oxide (Taketombo Co.)
Talc (Hayashi-Kasei Co., Ltd.)
Kaolin (Takehara Kagaku Kogyo Co., Ltd.)
The dry-coated tablets were produced according to the formulations of Table 3 in the same manner as in Experiment 1. The oxidant increase rates of the resulting dry-coated tablets after light irradiation were measured and changes in appearance of the tablets after light irradiation were observed in the same manner as in Test Example 1. Table 4 shows the results.

[Table 4]

| Sample | Increase (%) in Content of Oxidant after Light Irradiation | Change in Appearance |
|---|---|---|
| Example 4 | 0.0 | Not Observed |
| Example 5 | 0.1 | Not Observed |
| Example 6 | 1.0 | Slight Change observed |
| Example 7 | 0.2 | Not Observed |
| Example 8 | 0.1 | Not Observed |
| Example 9 | 0.7 | Not Observed |
| Example 10 | 0.1 | Not Observed |
| Comparative Example 5 | 9.4 | Not Observed |
| Comparative Example 6 | 3.5 | Not Observed |

The oxidant increase rate was calculated according to the following: (area in percentage of oxidant in the tablet after light irradiation) - (area in percentage of oxidant in the tablet before light irradiation).
Table 4 shows that the oxidant increase rate was reduced almost completely when incorporating only 0.1% Food Red No.2, Food Yellow No.4, Food Blue No.1, red ferric oxide, or black iron oxide in the outer layer of the dry-coated tablet. Particularly, the oxidant increase rate was reduced almost completely by incorporating only 0.01% red ferric oxide in the outer layer of the dry-coated tablet (Example 9). Further, regardless of the amount of nifedipine in the inner core, the oxidant increase rate of nifedipine was reduced almost completely by incorporating only 0.1% yellow ferric oxide in the outer layer of the dry-coated tablet (Example 10). On the other hand, talc and kaolin having light-blocking properties did not serve to reduce the increase in oxidant amount when they were incorporated in the outer layer of the dry-coated tablet in an amount of 10%.

[Experiment 3]

Tablet Formulation (mg)

[Table 5]

| | | Dry-Coated Tablets | | | | | | | |
|---|---|---|---|---|---|---|---|---|---|
| | | Example 11 | Example 12 | Example 13 | Example 14 | Example 15 | Example 16 | Example 17 | Example 18 |
| Thickness of Outer Layer | | 0.5 mm | 0.5 mm | 1.0 mm | 1.0 mm | 1.0 mm | 2.0 mm | 2.0 mm | 2.0 mm |
| Content in Outer Layer | | 0.05% | 0.10% | 0.025% | 0.05% | 0.10% | 0.025% | 0.05% | 0.10% |
| Content in Outer Layer per Surface Area of Inner Core ($\mu g/Mm^2$) | | 0.56 | 1.13 | 0.45 | 0.90 | 1.79 | 0.78 | 1.56 | 3.12 |
| Inner Core | D-Mannitol | 35.55 | | | | | | | |
| | Corn Starch | 3.95 | | | | | | | |
| | Nifedipine | 10 | | | | | | | |
| | St-Mq | 0.5 | | | | | | | |
| | Total | 50 | | | | | | | |
| Outer Layer | D-Mannitol | 75.70 | 75.66 | 120.25 | 120.22 | 120.16 | 209.33 | 209.28 | 209.17 |
| | Corn Starch | 8.41 | 8.41 | 13.36 | 13.36 | 13.35 | 23.26 | 23.25 | 23.24 |
| | Yellow ferric oxide | 0.043 | 0.085 | 0.034 | 0.068 | 0.135 | 0.059 | 0.118 | 0.235 |
| | St-Mq | 0.85 | 0.85 | 1.35 | 1.35 | 1.35 | 2.35 | 2.35 | 2.35 |
| | Total | 85 | 85 | 135 | 135 | 135 | 235 | 235 | 235 |
| Total | | 135 | 135 | 185 | 185 | 185 | 285 | 285 | 285 |

According to the formulations of Table 5, the dry-coated tablets were produced in the same manner as in Experiment 1. The oxidant increase rates of the obtained dry-coated tablets after light irradiation were measured and changes in appearance of the tablets after light irradiation were observed using the same method as in Test Example 1. Table 6 shows the results.

The thickness of the outer layer of each dry-coated tablet was adjusted by adjusting the amount of the outer-layer-forming powder.

[Table 6]

| Sample | Increase (%) in Content of Oxidant after Light Irradiation | Change in Appearance |
|---|---|---|
| Example 11 | 1.1 | Not Observed |
| Example 12 | 0.3 | Not Observed |
| Example 13 | 0.9 | Not Observed |
| Example 14 | 0.4 | Not Observed |
| Example 15 | 0.1 | Not Observed |
| Example 16 | 0.1 | Not Observed |
| Example 17 | 0.2 | Not Observed |
| Example 18 | 0.0 | Not Observed |

The oxidant increase rate was calculated according to the following: (area in percentage of oxidant in the tablet after light irradiation)-(area in percentage of oxidant in the tablet before light irradiation).

Table 6 revealed that, as well as the thickness of the outer layer and the concentration of yellow ferric oxide in the outer layer, the amount of the light-absorbing substance in the outer layer per surface area of the inner core influenced the photostability of nifedipine.

[Experiment 4]

Tablet formulation (mg)

[Table 7]

| | | General Tablet | | Dry-Coated Tablets | | | |
|---|---|---|---|---|---|---|---|
| | | Comparative Example 7 | Comparative Example 8 | Example 19 | Example 20 | Example 21 | Example 22 |
| Medicament | | Menaquinone -4 | Mecobalamin | Menaquinone -4 | Mecobalamin | Mecobalamin | Mecobalamin |
| Light-Absorbing Substance | | - | - | Yellow ferric oxide | Yellow ferric oxide | Red ferric oxide | Black Iron Oxide |
| Content in Outer Layer | | - | - | 0.1% | 1.0% | 1.0% | 0.1% |
| Content in Outer Layer per Surface Area of Inner Core ($\mu$g/Mm$^2$) | | - | - | 1.79 | 17.91 | 17.91 | 1.79 |
| Inner Core | D-Mannitol | 42.75 | 44.1 | 42.75 | 44.1 | 44.1 | 44.1 |
| | Corn Starch | 4.75 | 4.9 | 4.75 | 4.9 | 4.9 | 4.9 |
| | Medicament | 2 | 0.5 | 2 | 0.5 | 0.5 | 0.5 |
| | Yellow ferric oxide | - | - | - | - | - | - |
| | Magnesium Stearate | 0.5 | 0.5 | 0.5 | 0.5 | 0.5 | 0.5 |
| | Total | 50 | 50 | 50 | 50 | 50 | 50 |
| Outer Layer | D-Mannitol | - | - | 120.16 | 119.07 | 119.07 | 120.16 |
| | Corn Starch | - | - | 13.35 | 13.23 | 13.23 | 13.35 |
| | Light- Absorbing Substance | - | - | 0.135 | 1.35 | 1.35 | 0.135 |
| | Magnesium Stearate | - | - | 1.35 | 1.35 | 1.35 | 1.35 |
| | Total | - | - | 135 | 135 | 135 | 135 |
| Total | | 50 | 50 | 185 | 185 | 185 | 185 |

According to the formulations of Table 7, the dry-coated tablets were produced in the same manner as in Experiment 1 except that the final pressure in the molding of the dry-coated tablet was 6 kN.

Test Example 2

A photostability test was performed with respect to the tablets obtained in Comparative Examples 7, 8, and Examples 19 to 22.

The conditions used in light irradiation on the tablets are shown below. The tablets were irradiated at 20000 lux for 60 hours using a light source (xenon lamp) and a photostability tester (Model: LTX-01, Nagano Science Co., Ltd.).

After light irradiation, quantitative analysis of the decomposition product (related substance) of the medicament (menaquinone-4 or mecobalamin) in each tablet was performed by liquid chromatography (HPLC).

The changes in appearance of the tablets after light irradiation were examined by observing the changes in color by visual inspection. Table 8 shows the results.

The amount of generated related substance was measured as follows.

• Menaquinone-4

■ Method for preparing sample

Preparation of standard solution

    1. About 5 mg of menaquinone was precisely weighed and dispersed in 10 mL of water.
    2. Ethanol (99.5) was added to the dispersion until the total volume was exactly 50 mL, thereby preparing a standard solution (0.1 mg/mL).

Preparation of sample solution

    1. Each tablet was pulverized in a mortar and weighed the menaquinone content 1.0 mg in a 10 mL measuring flask.
    2. Water in the amount of 2 mL was added and ultrasonic treatment was performed for 5 minutes.
    3. After further adding ethanol (99.5), another ultrasonic treatment was performed for 5 minutes.
    4. Ethanol (99.5) was added until the total volume was exactly 10 mL (0.1 mg/mL) .
    5. Centrifugation was performed at 3000 rpm for 10 minutes.

■ HPLC analysis conditions

    Detector: Ultraviolet absorptiometer
    Column: L-column ODS (150 × 4.6 mm, 5 μm)
    Column temperature: 40°C
    Measurement wavelength: 270 nm
    Flow rate: 1.0 mL/min
    Injection volume: 50 μL
    Sample cooler: 25°C
    Retention time: About 7 minutes
    Syringe washing solution: Ethanol (99.5)
    Mobile phase: Methanol

• Mecobalamin

■ Method for preparing sample

Preparation of standard solution

1. About 20 mg of mecobalamin was precisely weighed. A sample-dissolving solvent (water) was added until the total volume was precisely 100 mL.
2. The resulting mixture in the amount of 5 mL was precisely weighed in a 20-mL measuring flask, and a sample-dissolving solvent (water) was added to a constant volume, thereby preparing a standard solution (50 μg/mL).

Preparation of sample solution

1. Each tablet was pulverized with a mortar and weighed until the mecobalamin content was 0.25 mg.
2. Sample-dissolving solvent in an amount of 5 mL was added, and ultrasonic treatment was performed.
3. The treated liquid was filtered with a filter (ADVANTEC DISMIC-25HP, PTFE 0.45 μm). 4 mL of the first filtrate was discarded and the rest was used as a sample solution (50 μg/mL).

■ HPLC analysis conditions

Detector: Ultraviolet absorptiometer
Column: Inertsil ODS-3 (150 × 4.6 mm, 3 μm)
Column temperature: 40°C
Measurement wavelength: 220 nm
Flow rate: 1.0 mL/min
Injection volume: 100 μL
Sample cooler: 20°C
Retention time: About 15 minutes
Syringe washing solution: Water
Mobile phase:

A: A solution obtained by dissolving sodium 1-heptanesulfonate in a mixed solution of 0.02 mol/L phosphoric acid/potassium dihydrogenphosphate buffer (a pH value = 2.6) and acetonitrile (21:4) at 2 mg/mL
B: Acetonitrile

[Table 8]

| Gradient Condition: High-Pressure Gradient Method | | |
| --- | --- | --- |
| Time (Min) | Solution A (%) | Solution B (%) |
| 0 | 90.0 | 10.0 |
| 10 | 80.0 | 20.0 |
| 40 | 70.0 | 30.0 |
| 60 | 5.0 | 95.0 |
| 70 | 5.0 | 95.0 |
| 75 | 90.0 | 10.0 |
| 90 | 90.0 | 10.0 |

[Table 9]

| Sample | Increase (%) in Content of related substance after Light Irradiation | Change in Appearance |
|---|---|---|
| Comparative Example 7 | 10.9 | Significant Change observed |
| Comparative Example 8 | 14.8 | Observed |
| Example 19 | 0.1 | Not Observed |
| Example 20 | 1.4 | Not Observed |
| Example 21 | 0.0 | Not Observed |
| Example 22 | 0.1 | Not Observed |

The related substance increase rate was calculated according to the following: (area in percentage of related substance in the tablet after light irradiation) - (area in percentage of related substance in the tablet before light irradiation) .

Table 9 revealed that the related substance increase can be suppressed almost completely not only in nifedipine but also in other medicaments unstable to light by incorporating a light-absorbing substance, such as yellow ferric oxide, red ferric oxide, or black iron oxide, in the outer layer of a dry-coated tablet.

As described above, the present invention makes it possible to provide an orally disintegrating tablet that ensures improved photostability of a medicament unstable to light by only incorporating a slight amount of a light-absorbing substance in the outer layer of an orally disintegrating tablet having an inner core.

The documents disclosed in the specification of the present invention are incorporated by reference.

Industrial Applicability

The present invention makes it possible to provide an orally disintegrating tablet that ensures improved photostability of a medicament unstable to light. Thereby, the present invention improves the quality of an orally disintegrating preparation that contains a medicament unstable to light, and the resulting preparation can be packed in a simpler and more easily portable way. This enables elderly people or busy working people to more easily carry the tablets and easily take the orally disintegrating preparation of the medicament unstable to light in any place without water.

**Claims**

1. An orally disintegrating tablet comprising an inner core and an outer layer that covers a surface of the inner core, wherein the inner core contains a medicament unstable to light and the outer layer contains a light-absorbing substance.

2. The orally disintegrating tablet according to claim 1, wherein the light-absorbing substance is at least one member selected from the group consisting of Food Red No.2, Food Red No.3, Food Red No.102, Food Red No.104, Food Red No.105, Food Red No.106, Food Yellow No.4, Food Yellow No.5, Food Green No.3, Food Blue No.1, Food Blue No.2, Food Red No.3 aluminum lake, Food Yellow No.4 aluminum lake, Food Yellow No.5 aluminum lake, Food Blue No.1 aluminum lake, Food Blue No.2 aluminum lake, red ferric oxide, yellow ferric oxide, black iron oxide, yellow oxide of iron, carmine, sodium copper chlorophyllin, copper chlorophyll, and red iron oxide.

3. The orally disintegrating tablet according to claim 1, wherein the light-absorbing substance is at least one member selected from the group consisting of Food Red No.2, Food Red No.3, Food Red No.102, Food Red No.104, Food Red No.105, Food Red No.106, Food Yellow No.4, Food Yellow No.5, Food Green No.3, Food Red No.3 aluminum lake, Food Yellow No.4 aluminum lake, Food Yellow No.5 aluminum lake, red ferric oxide, yellow ferric oxide, black iron oxide, yellow oxide of iron, carmine, sodium copper chlorophyllin, copper chlorophyll, and red iron oxide.

4. The orally disintegrating tablet according to any one of claims 1 to 3, wherein the light-absorbing substance is at least one member selected from the group consisting of red ferric oxide, yellow ferric oxide, and black iron oxide.

5. The orally disintegrating tablet according to any one of claims 1 to 4, wherein the outer layer contains the light-absorbing substance in an amount of 0.01 to 600 $\mu$g/=mm$^2$ per unit surface area of the inner core.

6. The orally disintegrating tablet according to any one of claims 1 to 4, wherein the outer layer contains the light-absorbing substance in an amount of 0.1 to 150 $\mu$g/=mm$^2$ per unit surface area of the inner core.

7. The orally disintegrating tablet according to any one of claims 1 to 4, wherein the outer layer contains the light-absorbing substance in an amount of 0.2 to 20 $\mu$g/=mm$^2$ per unit surface area of the inner core.

8. The orally disintegrating tablet according to any one of claims 1 to 4, wherein the outer layer contains the light-absorbing substance in an amount of 1.5 to 10 $\mu$g/=mm$^2$ per unit surface area of the inner core.

9. A method for improving photostability of a medicament unstable to light contained in an orally disintegrating tablet, the method comprising:

forming the orally disintegrating tablet so that the orally disintegrating tablet comprises an inner core and an outer layer that covers the surface of the inner core;
incorporating the medicament into the inner core; and
incorporating a light-absorbing substance into the outer layer.

10. A method for improving photostability of a medicament unstable to light contained in an orally disintegrating tablet that comprises an inner core containing the medicament and an outer layer that covers the surface of the inner core, the method comprising incorporating a light-absorbing substance into the outer layer.

11. A method for stabilizing a medicament unstable to light, the method comprising coating a tablet containing the medicament with an outer layer containing a light-absorbing substance.

| INTERNATIONAL SEARCH REPORT | International application No. |
|---|---|
| | PCT/JP2010/051294 |

**A. CLASSIFICATION OF SUBJECT MATTER**
*A61K9/30*(2006.01)i, *A61K47/02*(2006.01)i, *A61K47/20*(2006.01)i, *A61K47/22*
(2006.01)i, *A61K47/46*(2006.01)i

According to International Patent Classification (IPC) or to both national classification and IPC

**B. FIELDS SEARCHED**

Minimum documentation searched (classification system followed by classification symbols)
A61K9/30, A61K47/02, A61K47/20, A61K47/22, A61K47/46

Documentation searched other than minimum documentation to the extent that such documents are included in the fields searched
Jitsuyo Shinan Koho      1922–1996    Jitsuyo Shinan Toroku Koho    1996–2010
Kokai Jitsuyo Shinan Koho    1971–2010    Toroku Jitsuyo Shinan Koho    1994–2010

Electronic data base consulted during the international search (name of data base and, where practicable, search terms used)
CA/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| Y | WO 03/028706 A1 (Sanwa Kagaku Kenkyusho Co., Ltd.), 10 April 2003 (10.04.2003), entire text & US 2005/0202082 A1    & EP 1430888 A1 & CA 2460894 A | 1–11 |
| Y | WO 2007/052592 A1 (Kowa Co., Ltd.), 10 May 2007 (10.05.2007), entire text & US 2009/0041843 A1    & EP 1944029 A1 & CA 2626309 A | 1–11 |
| Y | JP 04-346929 A (Takata Seiyaku Co., Ltd.), 02 December 1992 (02.12.1992), entire text (Family: none) | 1–11 |

☒   Further documents are listed in the continuation of Box C.      ☐   See patent family annex.

| | |
|---|---|
| *   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| 22 February, 2010 (22.02.10) | 02 March, 2010 (02.03.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/051294

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-104887 A  (Maruko Seiyaku Kabushiki Kaisha), 09 April 2003 (09.04.2003), entire text (Family: none) | 1-11 |
| Y | JP 58-109415 A  (Teysan Pharmaceuticals Co., Ltd.), 29 June 1983 (29.06.1983), entire text (Family: none) | 1-11 |
| Y | JP 48-28621 A  (BAYER AG.), 16 April 1973 (16.04.1973), entire text & US 3784684 A          & GB 1362627 A & DE 2209526 A          & FR 2150848 A | 1-11 |
| Y | JP 55-22645 A  (FUJISAWA PHARM CO., LTD.), 18 February 1980 (18.02.1980), entire text (Family: none) | 1-11 |

Form PCT/ISA/210 (continuation of second sheet) (July 2009)

**REFERENCES CITED IN THE DESCRIPTION**

**Patent documents cited in the description**

- JP 55022645 A
- JP 2003104888 A
- JP 2000007583 A
- JP 2003028706 A
- JP 2007091648 A